# DEMANDE DE BREVET EUROPEEN

(11) **EP 1 304 370 A1**
(43) Date de publication de la demande: **23.04.2003**
(21) Numéro de dépôt: 01811030.4
(22) Date de dépôt: 22.10.2001
(51) Int. Cl.: C12M 3/04, C12M 1/10

(54) **Insert pour flacon rotatif**

(71) Demandeur: Integra Biosciences Holding AG, 6274 Eschenbach (CH)
(72) Inventeur: Macaluso, Joseph Nicola, 3325 Hettiswil (CH); Heyraud, Marc, 2300 La Chaux-de-Fonds (CH)
(74) Mandataire: BOVARD AG - Patentanwälte

(57) **Abrégé**

Insert (1) pour récipient (2) destiné à contenir un fluide (3), ce récipient (2), étant constitué par une paroi (20),
. munie d'une ouverture (22), notamment, obturable, et
. présentant un axe privilégié de rotation (21) et une surface interne dont au moins une partie est dite surface réceptrice (25), parce que placée au contact dudit fluide (3) lors de la rotation du récipient (2) autour dudit axe privilégié de rotation (21),
ledit insert (1) étant destiné à être disposé dans le récipient (2) et comprenant au moins un élément (101) qui présente au moins une surface (100) également destinée à être placée au contact du fluide (3) lors de la rotation dudit récipient (2) autour de l'axe privilégié de rotation (21),
cet insert (1) étant caractérisé en ce, lorsqu'il a été disposé dans le récipient (2), il présente au moins une telle surface (100) qui, destinée à être placée au contact du fluide (3) :
- est au moins localement tangente à un plan (26) sécant à l'axe privilégié de rotation (21), et
- s'étend, d'une part, au-delà de la surface réceptrice (25) du récipient (2) et, d'autre part, selon au moins une partie de la section transversale de ce récipient (2), et ce, sur une fraction (260) de la dimension transversale (200) de ce récipient (2).

## Description

L'invention se rapporte à un insert pour un récipient destiné à contenir un fluide dans lequel ledit insert doit être régulièrement immergé, tel un insert de support de cellules de culture.

Lorsqu'il est nécessaire de cultiver des cellules en milieu humide, on utilise classiquement des récipients allongés qui sont sensiblement cylindriques de révolution et comprennent deux extrémités opposées dont une présente une ouverture de section réduite par rapport à la section transversale du récipient, cette ouverture étant obturable, par exemple au moyen d'un bouchon de type vissant.

Au moins une partie de la surface interne du récipient, dite surface réceptrice, constitue une surface utilisable pour la fixation des cellules de culture.

En apparence, ces récipients sont semblables à des bouteilles.

Lors de leur utilisation, les récipients sont mis en rotation lente autour de leur axe longitudinal de manière telle que des cellules adhérant à la surface dite réceptrice soient régulièrement immergées dans un fluide nutritif contenu dans une partie inférieure de ces récipients.

De manière à accroître l'étendue de la surface du récipient utilisable pour la fixation de cellules et donc la capacité d'accueil de ce récipient, il est connu d'y placer un dispositif dit insert qui présente au moins une surface supplémentaire sur laquelle les cellules peuvent adhérer.

A cet effet, on connaît un insert US-A 3,941,661 constitué par une feuille de matériau enroulée sur elle-même en spirale, et ce, avec un espace entre ses spires.

L'enroulement spiralé est réalisé avec des dimensions externes qui sont voisines des dimensions internes du récipient de culture et de manière telle qu'il puisse être temporairement contracté sur lui-même autour de son axe longitudinal afin d'être axialement introduit dans le récipient, par l'ouverture de ce dernier.

Ce type d'insert présente une très grande surface utilisable pour la fixation de cellules mais, notamment, il ne garantit pas toujours la parfaite immersion de toute cette surface dans le fluide placé dans le récipient.

Un résultat que l'invention vise à obtenir est un insert du type précité, qui présente une surface utilisable pour la fixation de cellules qui est d'étendue accrue et garantit l'immersion des cellules qui y sont fixées.

A cet effet, l'invention a pour objet un insert pour récipient destiné à contenir un fluide, ce récipient, étant constitué par une paroi,
. munie d'une ouverture, notamment, obturable, et
. présentant un axe privilégié de rotation et une surface interne dont au moins une partie est dite surface réceptrice, parce que placée au contact dudit fluide lors de la rotation du récipient autour dudit axe privilégié de rotation,
ledit insert étant destiné à être disposé dans le récipient et comprenant au moins un élément qui présente au moins une surface également destinée à être placée au contact du fluide lors de la rotation dudit récipient autour de l'axe privilégié de rotation,
cet insert étant caractérisé en ce, lorsqu'il a été disposé dans le récipient, il présente au moins une telle surface qui, destinée à être placée au contact du fluide :
- est au moins localement tangente à un plan sécant à l'axe privilégié de rotation, et
- s'étend, d'une part, au-delà de la surface réceptrice du récipient et, d'autre part, selon au moins une partie de la section transversale de ce récipient, et ce, sur une fraction de la dimension transversale de ce récipient.

L'invention sera mieux comprise à la lecture de la description ci-après faite à titre d'exemple non limitatif en regard du dessin ci-annexé qui représente :
Figure 1 : vu en coupe transversale, un récipient équipé d'un insert selon l'invention,
Figure 2 : une vue en perspective d'un insert destiné à être mis en place dans un récipient, lors de la fabrication de ce récipient,
Figures 3 à 6 : vu en perspective et partiellement en coupe, un récipient équipé d'un insert selon l'invention dans différentes variantes de réalisation de cet insert,

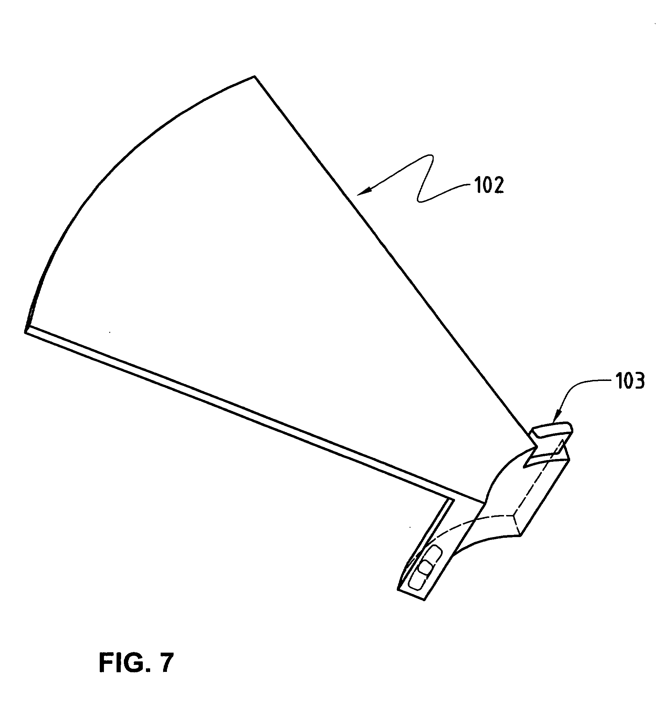

Figure 7 : une partie constitutive d'un insert selon l'invention dans une forme de réalisation.
Figure 8 : vus en perspective et en coupe partielle, un récipient et un insert conformé pour être introduit dans ce récipient.
Figure 9 : vus en perspective et en coupe partielle, un récipient et un insert en voie d'être introduit dans ce récipient,
Figure 10 : vu en perspective et partiellement en coupe, un récipient équipé d'un insert selon les figures 8 et 9.

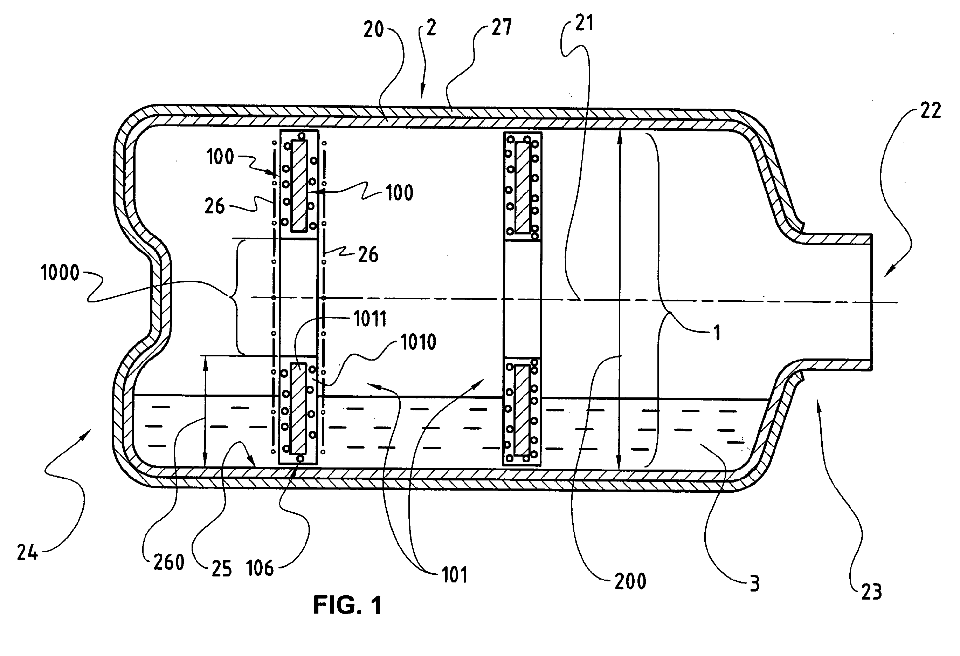

En se reportant au dessin, par exemple à la figure 1, on voit un insert 1 pour un récipient 2 destiné à contenir un fluide 3.

Le récipient 2 est constitué par une paroi 20 qui présente au moins un axe privilégié de rotation 21, et est munie d'une ouverture 22, notamment, obturable.

Le récipient 2 est allongé, sensiblement cylindrique de révolution et comprend deux extrémités opposées 23, 24 dont une présente l'ouverture 22, laquelle est de section réduite par rapport à la section transversale du récipient 2.

Par exemple, cette l'ouverture 22 est obturable, au moyen d'un bouchon de type vissant ou autre.

Le récipient 2 présente une surface interne dont au moins une partie est dite surface réceptrice 25, parce que placée au contact dudit fluide 3 lors de la rotation du récipient 2 autour dudit axe privilégié de rotation 21.

Tel que cela apparaît, l'insert 1 présente également au moins une surface 100 destinée à entrer en contact avec le fluide 3, notamment, lors de la rotation dudit récipient 2 autour de l'axe privilégié de rotation 21.

Par exemple, l'insert 1 est destiné à constituer un support de cellules de culture.

La surface réceptrice 25 du récipient 2, ainsi qu'au moins une surface 100 que présente l'insert 1 constituent notamment des surfaces utilisables pour la fixation des cellules de culture.

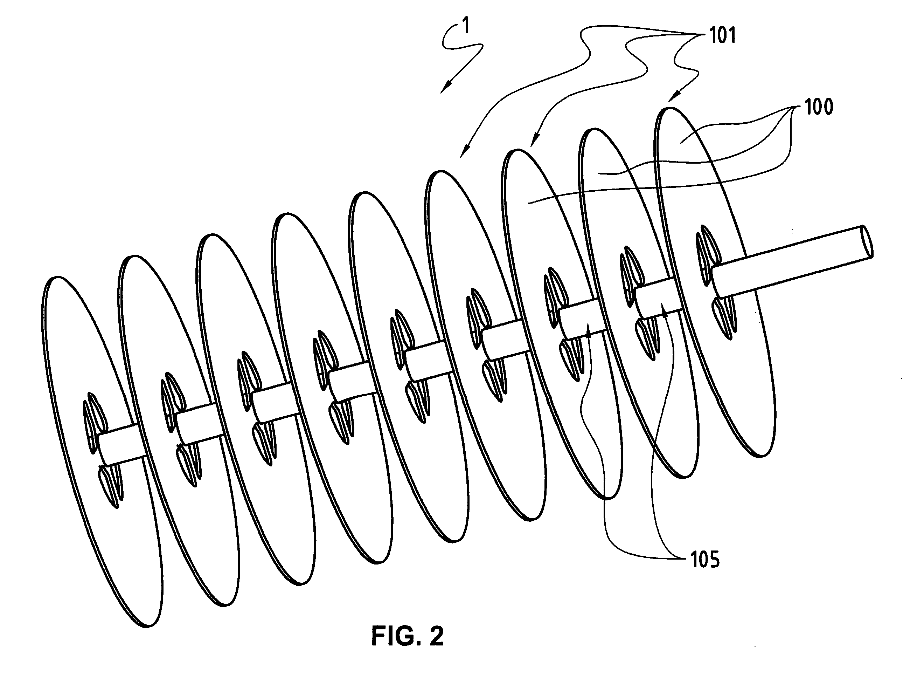

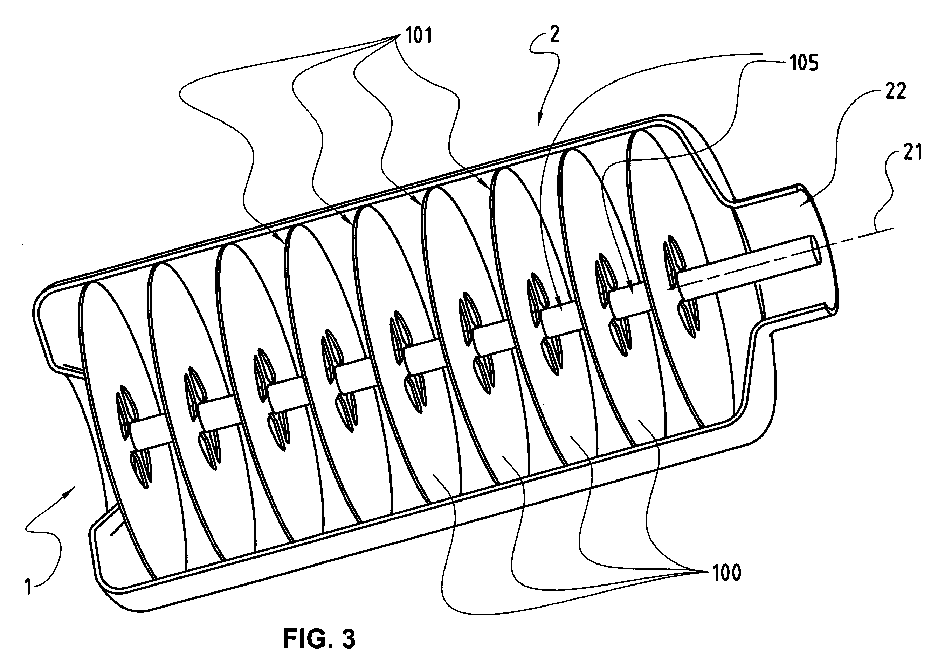

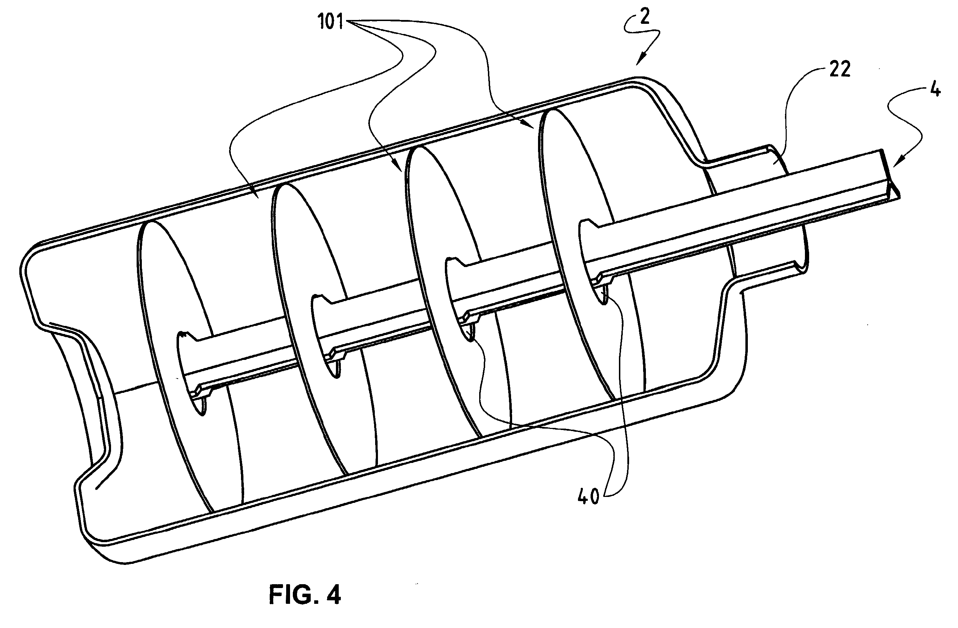

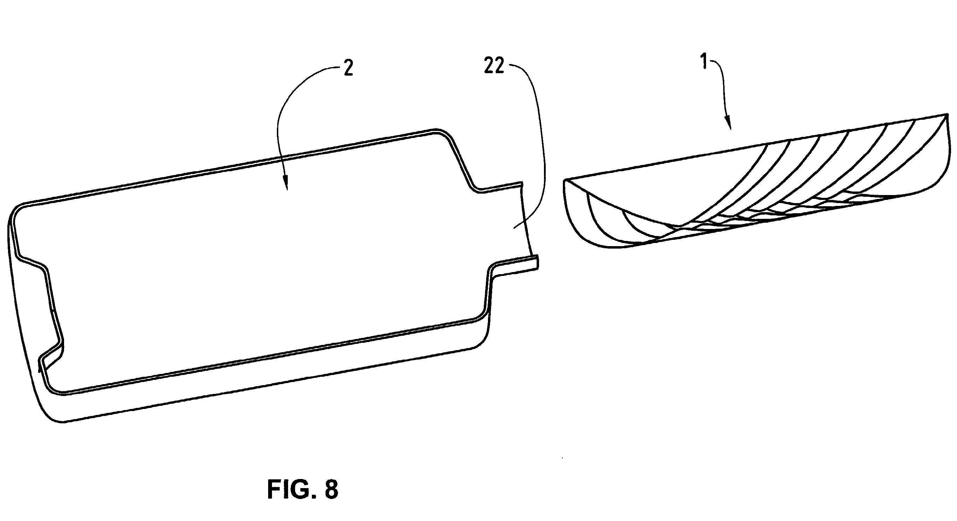

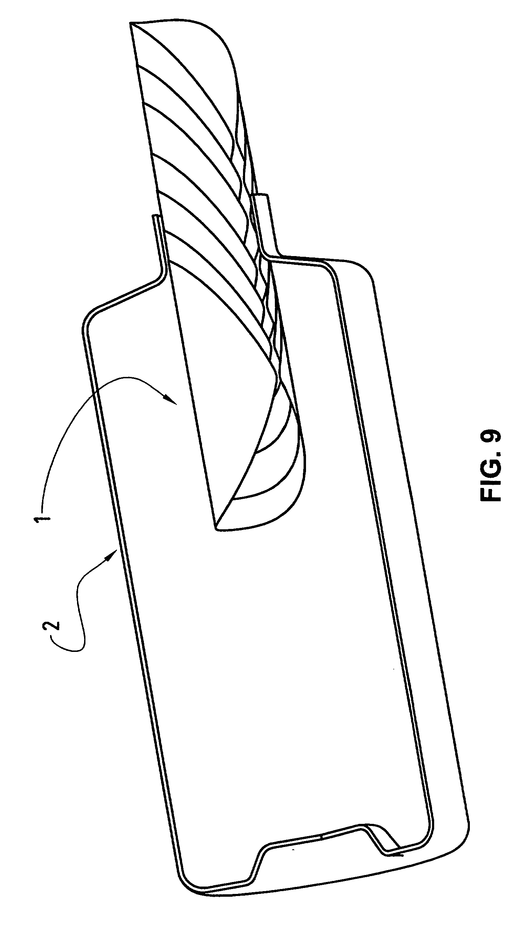

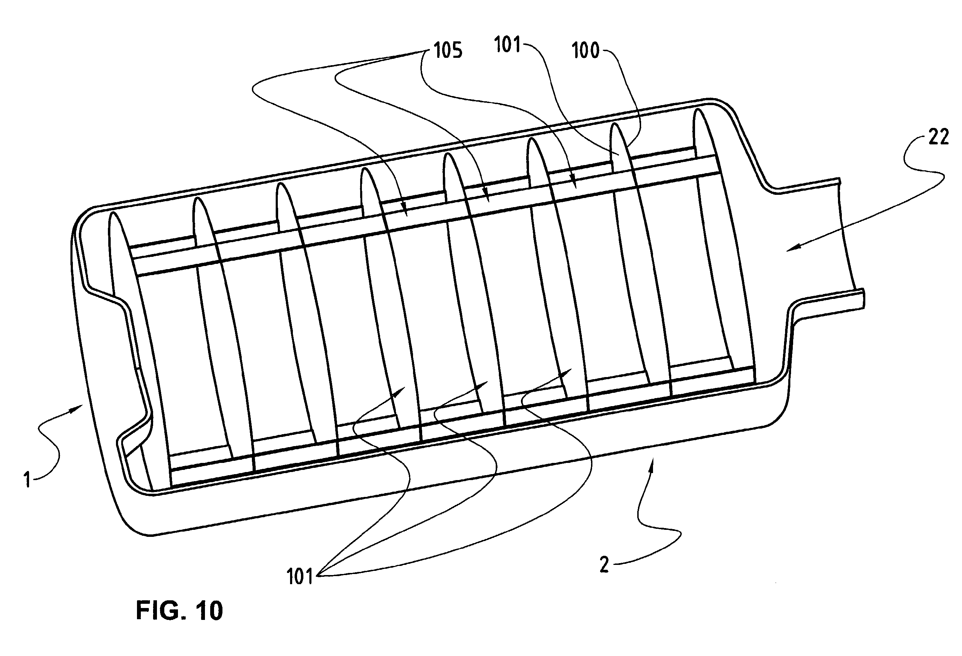

De manière remarquable, lorsqu'il a été disposé dans le récipient 2, l'insert 1 présente au moins une telle surface 100 qui, destinée à être placée au contact du fluide 3 :
- est au moins localement tangente à un plan 26 sécant à l'axe privilégié de rotation 21, et
- s'étend, d'une part, au-delà de la surface réceptrice 25 du récipient 2 et, d'autre part, selon au moins une partie de la section transversale de ce récipient 2, et ce, sur une fraction 260 de la dimension transversale 200 de ce récipient 2.
   En présentant au moins une surface 100 qui est au moins localement tangente à un plan 26 sécant à l'axe privilégié de rotation 21, par exemple une surface qui s'étend dans un plan transversal au récipient 2, l'insert de l'invention garantit notamment la libre rotation du récipient 2 autour de son axe privilégié de rotation 21, l'immersion effective des surfaces 100 de l'insert 1 destinée à supporter des cellules (du fait qu'aucune autre surface n'entrave le contact avec le fluide 3).
   Selon une première forme notable de réalisation, l'insert 1 comprend au moins un élément 101 mis en place dans le récipient 2 lors de la fabrication de ce dernier (voir, par exemple, les figures 2 et 3).
   Cette solution est avantageuse, lorsque le récipient comprend des extrémités opposées 23, 24 qui sont constituées par des pièces rapportées sur une partie tubulaire (solution non représentée).
   Selon une deuxième forme notable de réalisation, l'insert 1 comprend au moins un élément 101 qui peut être configuré (voir par exemple les figures 8 à 10) de manière,
- à être introduit dans le récipient 2 à travers l'ouverture 22 qu'il comprend et,
- à être disposé dans ledit récipient 2 avec au moins une surface 100 qui, d'une part, est au moins localement tangente à un plan 26 sécant à l'axe privilégié de rotation 21, et, d'autre part, s'étend sur une fraction de la section de ce récipient 2, considérée transversalement à cet axe 21.
   Le terme configuré qualifie au moins l'un des états que sont un état au moins partiellement déformable et un état au moins partiellement démontable.
   Selon une première version desdites première et deuxième forme de réalisation, l'insert 1 est constitué par au moins un élément 101 circulaire qui présente au moins une surface 100 destinée à entrer en contact avec le fluide 3, notamment, lors de la rotation dudit récipient 2 autour de son axe privilégié de rotation 21 (voir, par exemple; les figures 1 à 4).
   Suivant une deuxième version desdites première et deuxième forme de réalisation, l'insert 1 est constitué par au moins un élément 101 circulaire qui présente au moins une découpe 1000 sensiblement centrale (voir, par exemple, les figures 1 et 4).
   Selon une première variante de la deuxième forme de réalisation, l'insert 1 comporte au moins un élément 101 comprenant,
- au moins deux parties rigides qui ont chacune des formes et dimensions autorisant leur introduction dans le récipient 2 par l'ouverture 22 qu'il présente,
- au moins une pièce de liaison des deux parties rigides, laquelle pièce autorise le déplacement relatif desdites parties rigides au moins entre deux positions dont,
   . une première position dans laquelle les pièces sont disposées l'une par rapport à l'autre de manière à autoriser leur introduction dans le récipient 2, et
   . une deuxième position dans laquelle les pièces permettent la constitution d'une surface 100 qui, d'une part, est au moins localement tangente à un plan 26 sécant à l'axe privilégié de rotation 21, et, d'autre part, s'étend sur une fraction de la section de ce récipient 2, considérée transversalement à cet axe 21.

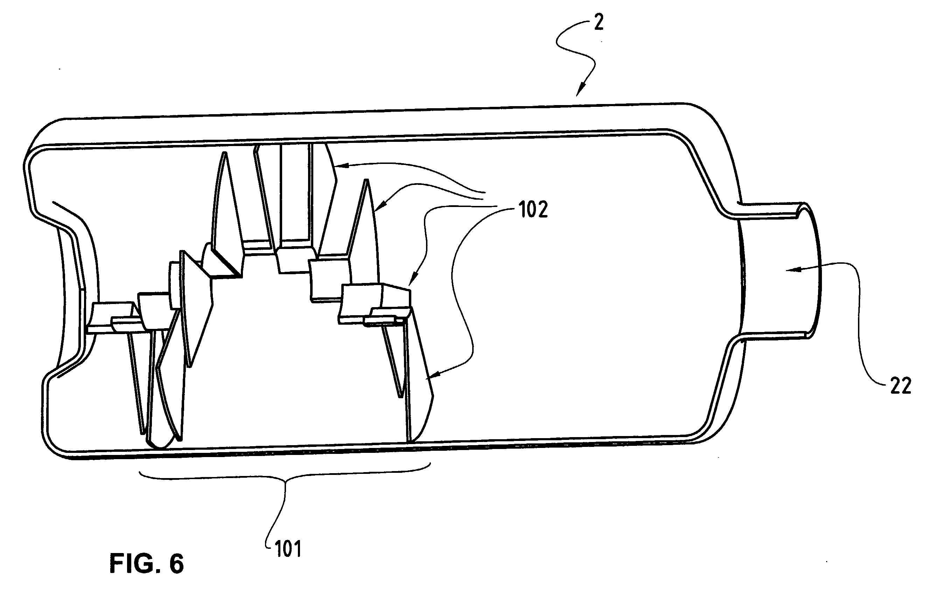

Selon une deuxième variante de la deuxième forme de réalisation, l'insert 1 comporte au moins un élément 101 comprenant,
- au moins deux parties rigides 102 qui ont chacune des formes et dimensions autorisant leur introduction dans le récipient 2 par l'ouverture 22 qu'il présente,
- au moins des portées d'assemblage 103, 104 des deux parties rigides 102, lesquelles portées autorisent,
   . la séparation desdites parties rigides 102 de manière à autoriser leur introduction dans le récipient 2, et
   . la réunion et le maintien desdites parties rigides 102 entre elles d'une manière telle qu'elle constitue une surface 100 qui, d'une part, est au moins localement tangente à un plan 26 sécant à l'axe privilégié de rotation 21, et, d'autre part, s'étend sur une fraction de la section de ce récipient 2, considérée transversalement à cet axe 21 (voir, par exemple, la figure 6).

Selon cette deuxième variante de la deuxième forme de réalisation, les parties rigides 102 sont configurées en forme de secteur circulaire et équipées de portées d'assemblage 103, 104, avantageusement de type par emboîtement, de manière telle qu'une fois assemblées ces dites parties rigides soient disposées d'une façon comparable aux marches d'un escalier hélicoïdal du type à marches rayonnantes.

Selon une troisième variante de la deuxième forme de réalisation, l'insert comporte au moins un élément 101 constitué d'au moins une pièce flexible 101, c'est à dire une pièce 101 qui,
- peut être déformée de manière à autoriser son introduction dans le récipient 2, et
- peut retrouver élastiquement une forme dans laquelle elle présente une surface 100 qui, d'une part, est au moins localement tangente à un plan 26 sécant à l'axe privilégié de rotation 21, et, d'autre part, s'étend sur une fraction de la section de ce récipient 2, considérée transversalement à cet axe 21 (voir, par exemple, les figures 1, 3, 4 et 10).

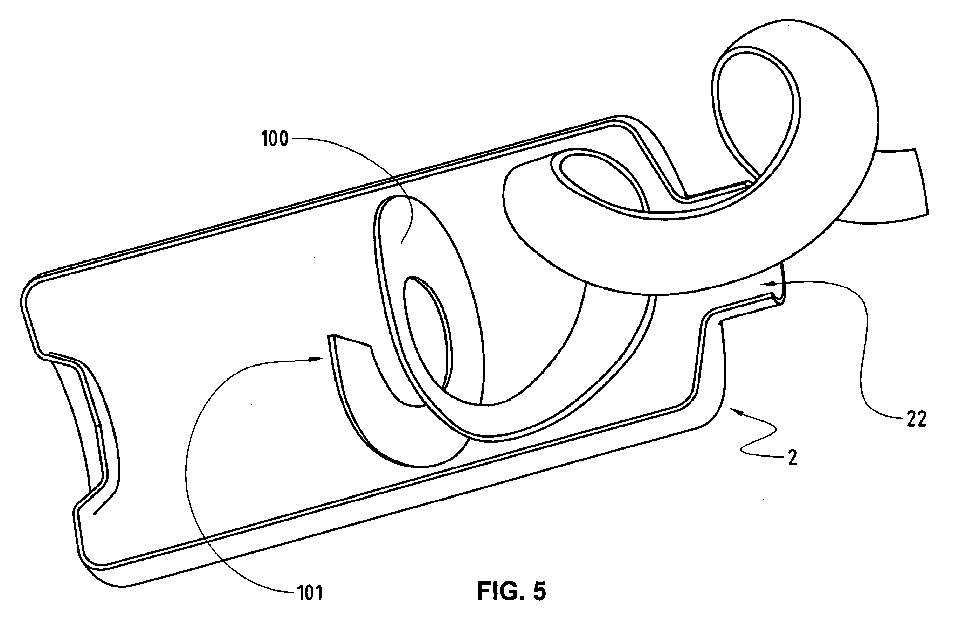

Conformément à une première version de cette troisième variante de la deuxième forme de réalisation, l'insert 1 est constitué par au moins un élément 101 allongé et plat, d'une part, d'une largeur au moins égale à une fraction de la dimension interne transversale du récipient 2, d'autre part, qui forme une spire hélicoïdale dont les sections transversales sont destinées à s'étendre sensiblement transversalement audit récipient 2 (voir, par exemple, la figure 5).

Avantageusement, la largeur de l'élément 101 allongé et plat est ajustée de manière telle que cet élément peut être introduit dans le récipient par vissage à travers son ouverture 22 (voir, par exemple, la figure 5).

Suivant une deuxième version de cette troisième variante de la deuxième forme de réalisation la pièce flexible 101 est constituée d'au moins un corps souple 1010 et d'au moins un corps flexible 1011 qui sont associés.

Conformément à une première solution de cette deuxième version, l'insert 1 est au moins partiellement réalisé en matière durcissable par exposition à un agent actif prédéterminé.

Selon une deuxième solution de cette deuxième version, l'insert 1 est au moins partiellement réalisé en matière durcissable par exposition à un rayonnement lumineux.

Suivant un premier mode de construction particulier des première et deuxième formes de réalisation précitées, l'insert comprend au moins deux éléments 101 qui sont maintenus espacés par au moins une pièce de liaison 105 constituant une entretoise 105.

Selon un deuxième mode de construction particulier des première et deuxième formes de réalisation précitées, l'insert 1 comprend des portées 106 d'appui contre la surface réceptrice 25 du récipient 2.

Conformément à un troisième mode de construction particulier des première et deuxième formes de réalisation précitées, l'insert 1 comprend des portées 106 d'appui et de fixation contre la surface réceptrice 25 du récipient 2.

Dans ce cas :
- d'une première manière remarquable les portées 106 d'appui et de fixation sont des portées 106 d'appui et de fixation par collage, ou
- d'une deuxième manière remarquable les portées 106 d'appui et de fixation sont des portées 106 d'appui et de fixation par soudage selon une technique permettant d'obtenir la fusion locale des matériaux de l'insert 1 et de la paroi 20, depuis l'extérieur de ladite paroi 20 du récipient 2.

L'invention concerne également un outil 4 pour la mise en place dans un récipient 2 d'un insert selon la deuxième forme de réalisation, cet outil 4 étant remarquable en ce qu'il comprend des portées 40 dites de maintien destinées à coopérer avec au moins un élément 101 de l'insert 1 pour,
- le maintenir dans la configuration qui permet de l'introduire dans le récipient 2 à travers l'ouverture 22 qu'il comprend et,
- le disposer dans ledit récipient 2 avec au moins une surface 100 qui, d'une part, est au moins localement tangente à un plan 26 sécant à l'axe privilégié de rotation 21, et, d'autre part, s'étend sur une fraction de la section de ce récipient 2, considérée transversalement à cet axe 21,
- le maintenir en cette configuration au moins jusqu'à ce que cette dernière soit stable.

L'invention se rapporte de manière générale à un récipient 2 équipé d'un insert 1 du type décrit plus avant que cet insert soit ou non mis en place au moyen d'un outil 4 tel que décrit.

L'invention se rapporte aussi à un récipient 2 équipé d'un insert 1 comprenant des portées 104 d'appui et de fixation contre la surface réceptrice 25 du récipient 2, ces portées étant des portées 106 d'appui et de fixation par soudage selon une technique permettant d'obtenir la fusion locale des matériaux de l'insert 1 et de la paroi 20, depuis l'extérieur de ladite paroi 20 du récipient 2.

Ce récipient est remarquable en ce que sa paroi 20 est étroitement recouverte d'une enveloppe étanche 27.

Cette particularité permet de garantir l'étanchéité de la paroi, notamment après que des soudures aient été réalisées.

L'invention s'applique avantageusement à l'équipement d'un récipient 2 destiné à la culture de cellules.

## Revendications

1. Insert (1) pour récipient (2) destiné à contenir un fluide (3), ce récipient (2), étant constitué par une paroi (20),
. munie d'une ouverture (22), notamment, obturable, et
. présentant un axe privilégié de rotation (21) et une surface interne dont au moins une partie est dite surface réceptrice (25), parce que placée au contact dudit fluide (3) lors de la rotation du récipient (2) autour dudit axe privilégié de rotation (21),
ledit insert (1) étant destiné à être disposé dans le récipient (2) et comprenant au moins un élément (101) qui présente au moins une surface (100) également destinée à être placée au contact du fluide (3) lors de la rotation dudit récipient (2) autour de l'axe privilégié de rotation (21),
cet insert (1) étant caractérisé en ce, lorsqu'il a été disposé dans le récipient (2), il présente au moins une telle surface (100) qui, destinée à être placée au contact du fluide (3) :
- est au moins localement tangente à un plan (26) sécant à l'axe privilégié de rotation (21), et
- s'étend, d'une part, au-delà de la surface réceptrice (25) du récipient (2) et, d'autre part, selon au moins une partie de la section transversale de ce récipient (2), et ce, sur une fraction (260) de la dimension transversale (200) de ce récipient (2).

2. Insert selon la revendication 1, **caractérisé en ce qu'**il comprend au moins un élément (101) mis en place dans le récipient (2) lors de la fabrication de ce dernier.

3. Insert selon la revendication 1, **caractérisé en ce qu'**il comprend au moins un élément (101) qui peut être configuré de manière,
- à être introduit dans le récipient (2) à travers l'ouverture (22) qu'il comprend et,
- à être disposé dans ledit récipient (2) avec au moins une surface (100) qui, d'une part, est au moins localement tangente à un plan (26) sécant à l'axe privilégié de rotation (21), et, d'autre part, s'étend sur une fraction de la section de ce récipient (2), considérée transversalement à cet axe (21).

4. Insert selon la revendication 3, **caractérisé en ce qu'**il comporte au moins un élément (101) comprenant,
- au moins deux parties rigides qui ont chacune des formes et dimensions autorisant leur introduction dans le récipient (2) par l'ouverture (22) qu'il présente,
- au moins une pièce de liaison des deux parties rigides, laquelle pièce autorise le déplacement relatif desdites parties rigides au moins entre deux positions dont,
. une première position dans laquelle les pièces sont disposées l'une par rapport à l'autre de manière à autoriser leur introduction dans le récipient (2), et
. une deuxième position dans laquelle les pièces permettent la constitution d'une surface (100) qui, d'une part, est au moins localement tangente à un plan (26) sécant à l'axe privilégié de rotation (21), et, d'autre part, s'étend sur une fraction de la section de ce récipient (2), considérée transversalement à cet axe (21).

5. Insert selon la revendication 3, **caractérisé en ce qu'**il comporte au moins un élément (101) comprenant,
- au moins deux parties rigides (102) qui ont chacune des formes et dimensions autorisant leur introduction dans le récipient (2) par l'ouverture (22) qu'il présente,
- au moins des portées d'assemblage (103, 104) des deux parties rigides (102), lesquelles portées autorisent,
. la séparation desdites parties rigides (102) de manière à autoriser leur introduction dans le récipient (2), et
. la réunion et le maintien desdites parties rigides (102) entre elles d'une manière telle qu'elle constitue une surface (100) qui, d'une part, est au moins localement tangente à un plan (26) sécant à l'axe privilégié de rotation (21), et, d'autre part, s'étend sur une fraction de la section de ce récipient (2), considérée transversalement à cet axe (21).

6. Insert selon la revendication 3, **caractérisé en ce qu'**il comporte au moins un élément (101) constitué d'au moins une pièce flexible (101), c'est à dire une pièce (101) qui,
- peut être déformée de manière à autoriser son introduction dans le récipient (2), et
- peut retrouver élastiquement une forme dans laquelle elle présente une surface (100) qui, d'une part, est au moins localement tangente à un plan 26 sécant à l'axe privilégié de rotation (21), et, d'autre part, s'étend sur une fraction de la section de ce récipient (2), considérée transversalement à cet axe (21).

7. Insert selon la revendication 6, **caractérisé en ce que** la pièce flexible (101) est constituée d'au moins un corps souple (1010) et d'au moins un corps flexible (1011) qui sont associés.

8. Insert selon l'une des revendications 6 ou 7, **caractérisé en ce qu'**il est au moins partiellement réalisé en matière durcissable par exposition à un agent actif prédéterminé.

9. Insert selon la revendication 8, **caractérisé en ce qu'**il est au moins partiellement réalisé en matière durcissable par exposition à un rayonnement lumineux.

10. Insert selon l'une quelconque des revendications 1 à 9, **caractérisé en ce qu'**il comprend au moins deux éléments (101) qui sont maintenus espacés par au moins une pièce de liaison (105) constituant une entretoise (105).

11. Insert selon l'une quelconque des revendications 1 à 10, **caractérisé en ce qu'**il comprend des portées (106) d'appui contre la surface réceptrice (25) du récipient (2).

12. Insert selon l'une quelconque des revendications 1 à 10, **caractérisé en ce qu'**il comprend des portées (106) d'appui et de fixation contre la surface réceptrice (25) du récipient (2).

13. Insert selon la revendication 12, **caractérisé en ce que** les portées (106) d'appui et de fixation sont des portées (106) d'appui et de fixation par collage.

14. Insert selon la revendication 12, **caractérisé en ce que** les portées (106) d'appui et de fixation sont des portées (106) d'appui et de fixation par soudage selon une technique permettant d'obtenir la fusion locale des matériaux de l'insert (1) et de la paroi (20), depuis l'extérieur de ladite paroi (20) du récipient (2).

15. Insert selon l'une quelconque des revendications 1 à 14, **caractérisé en ce qu'**il est constitué par au moins un élément (101) circulaire qui présente au moins une surface (100) destinée à entrer en contact avec le fluide (3), notamment, lors de la rotation dudit récipient (2) autour de son axe privilégié de rotation (21).

16. Insert selon la revendication 15, **caractérisé en ce qu'**il est constitué par au moins un élément (101) circulaire qui présente au moins une découpe (1000) sensiblement centrale.

17. Insert selon l'une quelconque des revendications 1 à 14, **caractérisé en ce qu'**il est constitué par au moins un élément (101) allongé et plat, d'une part, d'une largeur au moins égale à une fraction de la dimension interne transversale du récipient (2), d'autre part, qui forme une spire hélicoïdale dont les sections transversales sont destinées à s'étendre sensiblement transversalement audit récipient (2).

18. Insert selon la revendication 17, **caractérisé en ce que** la largeur de l'élément (101) allongé et plat est ajustée de manière telle que cet élément peut être introduit dans le récipient par vissage à travers son ouverture (22).

19. Outil (4) pour la mise en place dans un récipient (2) d'un insert selon l'une des revendications 3 à 18, **caractérisé en ce qu'**il comprend des portées (40) dites de maintien, destinées à coopérer avec au moins un élément (101) de l'insert (1) pour,
- le maintenir dans la configuration qui permet de l'introduire dans le récipient (2) à travers l'ouverture (22) qu'il comprend et,
- le disposer dans ledit récipient (2) avec au moins une surface (100) qui, d'une part, est au moins localement tangente à un plan (26) sécant à l'axe privilégié de rotation (21), et, d'autre part, s'étend sur une fraction de la section de ce récipient (2), considérée transversalement à cet axe (21),
- le maintenir en cette configuration au moins jusqu'à ce que cette dernière soit stable.

20. Récipient (2) équipé d'un insert (1) selon la revendication 14, **caractérisé en ce que** sa paroi (20) est étroitement recouverte d'une enveloppe étanche (27).

21. Récipient (2) équipé d'un insert (1) selon l'une quelconque des revendications 1 à 18.

22. Récipient (2) équipé d'un insert (1) selon l'une quelconque des revendications 3 à 18, mis en place au moyen de l'outil (4) selon la revendication 15.

23. Récipient selon l'une des revendications 20 et 22 destiné à la culture de cellules.
